# EUROPÄISCHE PATENTSCHRIFT

(11) **EP 2 793 957 B1**
(45) Veröffentlichungstag und Bekanntmachung des Hinweises auf die Patenterteilung: **07.04.2021**
(21) Anmeldenummer: 12809804.3
(22) Anmeldetag: 19.12.2012
(51) Int. Cl.: A61L 2/08, B65B 55/02, B67C 7/00, H01J 33/02, H01J 37/30

(54) **VORRICHTUNG UND VERFAHREN ZUM STERILISIEREN VON BEHÄLTNISSEN MIT FUNKTIONSÜBERWACHUNG**
DEVICE AND METHOD FOR STERILISING CONTAINERS WITH FUNCTION MONITORING
DISPOSITIF ET PROCÉDÉ PERMETTANT DE STÉRILISER DES RÉCIPIENTS AVEC SURVEILLANCE DE FONCTIONNEMENT

(30) Priorität: 19.12.2011 DE 102011056628
(43) Veröffentlichungstag der Anmeldung: 29.10.2014
(73) Patentinhaber: Krones AG, 93073 Neutraubling (DE)
(72) Erfinder: KRÜGER, Jochen, 93073 Neutraubling (DE)
(74) Vertreter: Bittner, Bernhard
(86) Internationale Anmeldenummer: PCT/EP2012/076186
(87) Internationale Veröffentlichungsnummer: WO 2013/092735

(56) Entgegenhaltungen:
- EP-A1- 2 316 495
- EP-A1- 2 316 495
- WO-A1-2011/011079
- WO-A1-2011/011079
- JP-A- 2007 126 168
- JP-A- 2007 126 168
- WEISS D E ET AL: "Electron beam process validation for sterilization of complex geometries", RADIATION PHYSICS AND CHEMISTRY, ELSEVIER SCIENCE PUBLISHERS BV, AMSTERDAM NL, Bd. 63, Nr. 3-6, 1. März 2002 (2002-03-01) , Seiten 581-586, XP027403337, ISSN: 0969-806X [gefunden am 2002-03-01]
- WEISS D E ET AL: "Electron beam process validation for sterilization of complex geometries", RADIATION PHYSICS AND CHEMISTRY, PERGAMON, AMSTERDAM, NL, vol. 63, no. 3-6, 1 March 2002 (2002-03-01), pages 581-586, XP027403337, ISSN: 0969-806X [retrieved on 2002-03-01]

## Beschreibung

Die vorliegende Erfindung bezieht sich auf eine Vorrichtung und ein Verfahren zum Sterilisieren von Behältnissen mittels Elektronen.

Im Rahmen von Behandlungsanlagen und Verfahren zum Herstellen von Getränkebehältnissen ist die Sterilisation der Behältnisse (beispielsweise von Flaschen oder auch Kunststoffvorformlingen, welche zu Flaschen umgeformt werden) neben dem eigentlichen Füllvorgang der zentrale Prozessschritt in einer aseptischen Abfüllanlage. Bei der Entkeimung der Behältnisse mittels Elektronenstrahlen ist zum einen die Sicherheit der Behandlung, d.h. die Sterilisationswirkung auf jeden einzelnen Behälter wichtig. Andererseits ist jedoch auch die Sicherheit der Bediener und des Personals der Anlage ein wesentlicher Aspekt.

In jüngerer Zeit ist man auch dazu übergegangen, die Behältnisse mittels Ladungsträgerstrahlung und insbesondere mittels Elektronenstrahlung zu beaufschlagen, um so eine Sterilisation zu erreichen. Bei der Bestrahlung der Behältnisse mit hoch energetischen Elektronen entsteht als unerwünschter Nebeneffekt auch Röntgenstrahlung. Diese Röntgenstrahlen entstehen, wenn die Elektronen abgebremst werden, z.B. an der Titanfolie des Austrittsfensters, aber auch, wenn sie auf die Behältnisse treffen. Um die Person und auch elektronische Komponenten in der Umgebung der Maschine vor dieser Strahlung zu schützen, werden Abschirmungen angebracht, die aus einem Material mit ausreichender Wandstärke bestehen. Für Elektronenstrahlen mit einer Energie im Bereich von 150 keV werden zur Abschirmung der Röntgenstrahlung Bleiwände mit einer Dicke im Bereich von 5-12 mm benötigt (je nach Stromstärke). Alternativ kann jedoch auch anderes Material, wie z.B. Stahl verwendet werden, jedoch ist die Abschirmwirkung von Stahl deutlich geringer, so dass die Wandstärke entsprechend dicker wird.

Üblicherweise ist jedoch die vollständige Anlage derart abgeschirmt, dass es bei den definierten Parametern der Strahlung und einem bestimmungsgemäßen, d.h. normalen Betrieb der Anlage an keiner Stelle außerhalb der Anlage zu einer Reststrahlung von mehr als 1 µSv/h kommen kann.

Es können jedoch auch (sicherheitsrelevante) Störfälle auftreten, wenn z.B. einer oder mehrere der Strahler defekt sind oder falsch angesteuert werden, so dass eine erhöhte Strahlung produziert wird.

Um diesen bestimmungsgemäßen oder normalen Betrieb sicherzustellen, sind Detektoren notwendig. Dies können beliebige Röntgen- oder Strahlendetektoren oder Dosimeter sein, die während dem Betrieb ein bestimmtes Strahlungsniveau innerhalb oder außerhalb der Maschine messen. Überschreitet dieses Messsignal einen vorgegebenen Grenzwert, so kann die maximale Reststrahlung außerhalb der Maschine überschritten werden, was zu einer Gefährdung des Bedienpersonals führt. Als Konsequenz muss die Anlage sofort abgeschaltet werden (Not-Aus). Wird andererseits ein vorgegebener Grenzwert unterschritten, so deutet dies auf den Ausfall oder auf eine Störung an einem oder mehreren Strahlern in der Anlage hin, mit der Folge, dass die Sterilisationswirkung auf die Behältnisse nicht mehr gewährleistet ist. Auch hier müssen Gegenmaßnahmen eingeleitet werden.

Aus der EP 2 316 495 A1 ist eine Sterilisationseinrichtung mit Elektronenstrahlern bekannt. Dabei ist eine stationär angeordnete Elektronenstrahleinrichtung vorgesehen, sowie ein bezüglich der Behältnisse gegenüberliegender Detektor, der die Elektronenstrahlen aufnimmt. Um eine derartige Störung erfassen zu können, werden Grenzwerte festgelegt, die oberhalb und unterhalb der jeweils aufgenommenen Strahlung liegen.

Die US 7,641,851 B2 beschreibt ein Verfahren und eine Vorrichtung zum Beurteilen eines Sterilisationsprozesses. Diesem Verfahren liegt die Aufgabe zu Grunde, die Sterilisation von medizinischen Produkten zu überwachen. Dabei wird eine Minimalsterilisationsdosis ermittelt, welche eine bestimmte gewünschte Sterilität erreicht. Danach werden diesem Elektronenstrahl eine Versuchskomponente und eine Vielzahl von Indikatoren zugeführt. Anschließend wird eine Strahlungsverteilung auf dieser Komponente überprüft. Dieses Verfahren ist relativ aufwendig und erfordert eine Vielzahl von Strahlungsdetektoren, um die Wirksamkeit der Bestrahlung zu überprüfen.

Auch aus der WO 00/71173 A2 sind eine Vorrichtung und ein Verfahren zum Schützen von Schaltkreisen während Sterilisationsprozessen bekannt.

Die JP 2007 126168 A1 Elektronenstrahl-Sterilisationsanlage umfasst eine Elektronenstrahl-Bestrahlungseinrichtung zum Bestrahlen eines von einer Fördereinrichtung zu transportierenden Lebensmittelbehälters mit Elektronenstrahlen, eine Speichereinheit zum Speichern der Bedingungen, unter denen die Steuerwerte wie Strahlstrom, Beschleunigungsspannung, Abtastfrequenz und Abtastbreite der Elektronenstrahl-Bestrahlungseinrichtung und die Fördergeschwindigkeit der Fördereinrichtung ausreichend sind, eine Bestimmungseinheit zur Bestimmung, ob die tatsächlichen Steuerwerte die Bedingung erfüllen oder nicht, und eine Entnahmevorrichtung zur Bestimmung, dass die Elektronenstrahlen der ausreichenden Bestrahlungsmenge den Lebensmittelbehälter nicht bestrahlen, wenn die Bedingung nicht erfüllt ist, und zur Entnahme des Lebensmittelbehälters aus einer Förderlinie.

Die EP2316495 A1 stellt einen Elektronenstrahl-Sterilisator zur Verfügung, der in einer Elektronenstrahl-Bestrahlungszone beurteilt, ob die Menge des Elektronenstrahls, mit der eine Harzflasche bestrahlt werden soll, angemessen oder unangemessen ist. Der Sterilisator enthält einen Elektronenstrahl-Bestrahler zum Bestrahlen der Harzflasche mit einem Elektronenstrahl durch ein Bestrahlungsfenster und einen Flaschenförderer zum Fördern der Harzflasche, und die vor das Bestrahlungsfenster geförderte Harzflasche wird mit dem Elektronenstrahl bestrahlt, um dadurch die Flasche zu sterilisieren. Ein Strahlkollektor ist vor dem Bestrahlungsfenster gegenüber dem Bestrahlungsfenster angeordnet. Der Strahlkollektor wird elektrisch isoliert von einem Isolierteil getragen. Wenn der Elektronenstrahl den Strahlkollektor bestrahlt, wird ein elektrischer Strom, der in den Strahlkollektor fließt, durch eine elektrische Strommesseinrichtung gemessen. Der gemessene elektrische Strom wird von einem Komparator mit einem vorbestimmten Referenzwert verglichen, und eine Beurteilungseinheit beurteilt, ob eine Menge des Elektronenstrahls, der auf die Harzflasche gestrahlt wird, richtig oder falsch ist. Bei der Beurteilung, ob sie als unzulässig beurteilt wird, werden die unzulässige Harzflasche und andere Harzflaschen, die vor und nach dieser Harzflasche befördert werden, von einer Ausscheideeinheit gemäß den Anweisungen einer Befehlseinheit ausgeschieden.

Die WO2011011079 (A1) bietet verbesserte Elektronenstrahl-Sterilisationsgeräte und Abschirmtechniken für den Einsatz in diesem Bereich. Eine Steuerung moduliert einen Elektronenstrahl beim Sterilisieren eines Innenraums eines Objekts, um sicherzustellen, dass eine angemessene Dosis erhalten wird. Sterilisationskarussells sind mit Eingangs-/Entladungszuführungen konfiguriert, um die Möglichkeit zu verringern, dass Menschen gefährlichen Strahlungsniveaus ausgesetzt werden. Das System reduziert die Menge der erforderlichen Abschirmung und senkt dadurch die Kosten für die Installation.

Das Dokument "WEISS D E ET AL"Electron beam process validation for sterilization of complex geometries", RADIATION PHYSICS AND CHEMISTRY, ELSEVIER SCIENCE PUB-LISHERS BV, AMSTERDAM NL, Bd. 63, Nr. 3-6,1. März 2002 (2002-03-01), Seiten 581-586, XP027403337, ISSN: 0969-806X [gefunden am 2002-03-01]" zeigt ebenso eine Elektronenstrahldesinfektionsvorrichtung.

Allgemein kann es jedoch auch bei den unterschiedlichen Messmethoden vorkommen, dass das Messsignal aufgrund einer Störung des Sensors bzw. Detektors einfriert und einen Messwert vortäuscht, der vermeintlich im sicheren Bereich ist. Aus diesem Grund werden in der Sicherheitstechnik dynamische Messtechniken eingesetzt, die neben einer bestimmten Messgröße auch eine periodische Schwankung messen und bewerten. Zu diesem Zweck werden die Messgrößen periodisch moduliert und der Sensor muss diesen Modulationen folgen.

Dieses Prinzip kann nun bei der Elektronenstrahlsterilisation zur Überwachung der Anlage gezielt eingesetzt werden. Es soll also nicht einfach nur ein Sensor außerhalb der Anlage installiert werden, der gewährleistet, dass das Strahlungsniveau außerhalb der Anlage unterhalb des erlaubten Grenzwertes bleibt, sondern der Detektor soll so platziert werden, dass er in jedem Fall ein deutliches Strahlungssignal misst. Bevorzugt wird er auch an einer Stelle platziert, in der starke oder allenfalls abgeschwächte Strahlung herrscht. Die Platzierung solch eines Sensors ist sehr schwierig und stellt keinesfalls sicher, dass nicht an einer anderen Stelle der Maschine Strahlung entweicht, die diesen Sensor nicht erreicht. Die Ausbreitung von Röntgenstrahlung aus einem kleinen Leck heraus (z.B. einem Türspalt oder einer unzureichenden Schweissnaht) ist im Wesentlichen geradlinig und kann in sehr hoher Stärke am Sensor vorbeigehen.

Dabei tritt jedoch das Problem auf, dass die oben erwähnten Detektorsignale schwanken und auch gewollt schwanken. Durch die in der oben erwähnten Druckschrift erwähnte Grenzwertbildung wird es daher erforderlich, dass die Grenzwerte zueinander einen sehr großen Abstand aufweisen müssen. Der Erfindung liegt daher die Aufgabe zu Grunde, diese Messung der auftretenden Strahlung zu verbessern bzw. genauer zu gestalten. Diese Aufgaben werden durch die Gegenstände der unabhängigen Ansprüche gelöst. Vorteilhafte Ausführungsformen und Weiterbildungen sind Gegenstand der Unteransprüche.

Bei einem erfindungsgemäßen Verfahren zum Betreiben einer Vorrichtung zum Sterilisieren von Behältnissen werden die Behältnisse mittels einer Transporteinrichtung entlang eines vorgegebenen Transportpfades transportiert und während des Transportes dieser Behältnisse wird mittels wenigstens einer ersten Strahlungseinrichtung wenigstens eine Wandung der Behältnisse mit einer Elektronenstrahlung bestrahlt. Weiterhin ist eine Sensoreinrichtung vorgesehen, welche Elektronenstrahlung oder von den Elektronen verursachte Strahlung erfasst und welche derart angeordnet ist, dass die auf die Sensoreinrichtung auftreffende Strahlung aufgrund der Bewegung der Behältnisse entlang des Transportpfades Schwankungen unterworfen ist, wobei diese Sensoreinrichtung wenigstens einen für die Strahlung charakteristischen Verlauf erfasst, und wenigstens ein erster Grenzwert definiert wird, und ein Vergleich zwischen diesem Grenzwert und dem Verlauf durchgeführt wird.

Erfindungsgemäß weist der Grenzwert einen (insb. definierten) - zeitlich und/oder örtlich - veränderlichen bzw. sich (insb. im Arbeitsbetrieb) verändernden Wert auf.

Die Anordnung der Sensoreinrichtung wird daher bewusst so gewählt, dass von dieser kein konstantes Signal aufgenommen wird, sondern dieses bedingt durch die Bewegung der Behältnisse schwankt. Falls sich die Strahlungseinrichtungen mit den Behältnissen mitbewegen, genügt es, die Sensoreinrichtung stationär anzuordnen, da sich die auf die Sensoreinrichtung auftreffende Strahlung bereits aufgrund der sich ändernden Abstände zwischen der

Strahlungseinrichtung und der Sensoreinrichtung ändern wird.

Im Gegensatz zum Stand der Technik wird daher nicht ein konstanter Grenzwert festgelegt, sondern dieser ändert sich. Vorteilhaft weist der Grenzwert einen zeitlichen und/oder ortsabhängigen Verlauf auf, der an den charakteristischen Verlauf der gemessenen Strahlung angepasst ist. So ist es möglich, dass beispielsweise im Rahmen eines Einlernbetriebes die für den Verlauf charakteristischen Schwankungen aufgenommen werden und auch der Grenzwert an diesen Verlauf angepasst wird. Bei einer vorteilhaften Ausführungsform ist die wenigstens eine Sensor- bzw. Detektoreinrichtung stationär angeordnet. Es wäre jedoch auch möglich, dass die Sensoreinrichtung mit den Behältnissen mitbewegt wird. Es wird daher eine orts- und/oder zeitabhängige Modulation des Grenzwerts vorgeschlagen.

Die Idee ist nun, den Sensor so zu platzieren, dass er durch die vorbeifahrenden Behälter ein zeitlich moduliertes Signal erfährt und auswertet.

Anhand der Form des Signals (Intensität und zeitlicher Verlauf) kann vorteilhaft im Vergleich mit einer angelernten Signalform entschieden werden, ob eine Störung vorliegt (z.B. dass ein Behälter in der Reihe fehlt, oder dass der Emitter zu starke oder zu schwache Strahlung aussendet).

Zur Redundanz der Messung und zur Überwachung beider (Flächen-)emitter können auch mehrere dieser Sensoren/Systeme in der Maschine verbaut werden, die jeweils eine unterschiedliche dynamische Signalform messen und auswerten.

Für die Innensterilisation der Behältnisse werden die Behältnisse vorteilhaft auf einem Karussell behandelt. Mehrere Strahlenemitter sind auf diesem Karussell installiert und das Behältnis wird relativ zum Emitter - insbesondere in einer Längsrichtung des Behältnisses - bewegt. Bei dem Behältnis kann es sich sowohl um Glas- oder Kunststoffflaschen als auch um Kunststoffvorformlinge, die in Kunststoffbehältnisse umformbar sind, handeln.

Die Strahlungsquelle, die hier festzustellende Strahlung emittiert, ist hier der Strahlfinger selbst bzw. dessen Titanfolie und/oder der Kunststoffvorformling bzw. das Kunststoffbehältnis, an dem die Elektronen gestreut werden.

Wird nun ein Sensor an einer fixen Stelle der Anlage in der Nähe des Behandlungskarussells platziert, so erhält dieser Sensor beim Vorbeifahren der einzelnen Stationen an diesem Sensor ein periodisch moduliertes Signal, dessen Intensität vom Stellungswinkel des Karussells abhängt. Die zeitliche Periodizität des Signals hängt damit vom Abstand und der Geschwindigkeit der vorbeifahrenden Strahler ab (Drehzahl des Karussells). Die Form des periodischen Signals kann von vielen Faktoren wie z. B. der Behälterform abhängen. Sie sollte aber während der Produktion und Behandlung von gleichartigen Behältern im Rahmen von Toleranzen für jeden Behälter gleich sein, vergleichbar mit einem Fingerabdruck.

Solange alle Strahler wie vorgesehen funktionieren, bewegt sich das Messsignal abhängig von der Messgenauigkeit und statistischen Schwankungen innerhalb von einem Toleranzkorridor. Hierzu kann das System, insbesondere mittels der oben beschriebenen Grenzwerte "angelernt" werden. Während einem nachgewiesenermaßen funktionierenden und sicheren Prozess wird die Form des Signals gemessen und abgespeichert. Während der nachfolgenden Produktion wird die gemessene Signalform (bevorzugt ständig) mit der gespeicherten Form verglichen. Dabei ist es vorteilhaft, für jede einzelne Behandlungsstation einen separaten Kurvenverlauf zu hinterlegen bzw. aufzuzeichnen, so dass im Falle eines Strahlerwechsels lediglich ein Kurvenverlauf trainiert und bezüglich der Empfindlichkeit parametriert werden muss.

Vorteilhaft sind wenigstens zwei Sensoreinrichtungen vorgesehen, welche jeweils die Strahlung bzw. einen für die Strahlung charakteristischen Verlauf erfassen. Dabei sind bevorzugt diese Sensoreinrichtungen derart angeordnet, dass die auf sie auftreffende Strahlung aufgrund der Bewegung der Behältnisse schwankt. Dabei beruhen wenigstens zwei derartiger Sensoreinrichtungen bevorzugt auf unterschiedlichen Messprinzipien. Auf diese Weise wird eine Redundanz für die Messung erreicht.

Bei einem vorteilhaften Verfahren wird der Grenzwert, wie oben erwähnt, an den für die Strahlung charakteristischen zeitlichen Verlauf angepasst.

Bei einem weiteren vorteilhaften Verfahren ist die Sensoreinrichtung derart angeordnet, dass zumindest Abschnitte der Behältnisse und/oder der die Behältnisse haltenden Halteeinrichtungen zwischen der Strahlungseinrichtung und der Sensoreinrichtung transportiert werden. Diese Variante ist besonders dann relevant, wenn sowohl die Strahlungseinrichtung als auch die Sensoreinrichtung stationär angeordnet sind. In diesem Fall wird die Schwankung der auf der Sensoreinrichtung insbesondere auch durch die sich ändernde Abschirmung der Strahlung durch die Behältnisse oder deren Halteeinrichtungen bewirkt.

Bei einem weiteren vorteilhaften Verfahren wird die wenigstens eine Strahlungseinrichtung mit den Behältnissen mitbewegt. Dabei ist es möglich, dass die Strahlungseinrichtung ebenfalls auf dem gleichen Träger ist, z.B. der Transporteinrichtung, welche auch die Behältnisse transportiert. Bei einem weiteren vorteilhaften Verfahren wird wenigstens ein Abschnitt der Strahlungseinrichtung in einen Innenraum der Behältnisse eingeführt. Die Strahlungseinrichtung kann dabei, wie an sich aus dem Stand der Technik bekannt, als Strahlfinger ausgebildet sein, der durch die Mündung der Behältnisse hindurch in das Innere der Behältnisse eingeführt wird. Eine derartige Vorgehensweise ist beispielsweise aus der 1 982 921 A1 bekannt.

Vorteilhaft wird wenigstens eine Außenoberfläche und wenigstens eine Innenoberfläche der Behältnisse mit Elektronenstrahlung beaufschlagt.

Vorteilhaft werden die Innenwandungen der Behältnisse mit einer Vielzahl von Strahlungseinrichtungen bestrahlt. So kann beispielsweise eine Vielzahl der oben erwähnten Strahlfinger eingesetzt werden, die in die unterschiedlichen Behältnisse eintauchen, um diese zu sterilisieren.

Bei einem weiteren vorteilhaften Verfahren kann durch den Vergleich zwischen dem Grenzwert und dem (gemessenen) Verlauf der Strahlung ermittelt werden, ob die Bestrahlung der Behältnisse vorgegebenen Kriterien genügt. Es wird hier darauf hingewiesen, dass auch die Elektronen selbst, obwohl es sich hier physikalisch um Teilchen handelt, auch als Strahlung aufgefasst werden.

Bei einem weiteren vorteilhaften Verfahren kann auch ein zweiter Grenzwert definiert werden und ein Vergleich zwischen diesem zweiten Grenzwert und dem Verlauf durchgeführt werden. So kann beispielsweise ein Maximalgrenzwert definiert werden, sowie auch ein Minimalgrenzwert und dabei kann bestimmt werden, dass der gemessene Verlauf stets bei einem durch diese beiden Grenzwerte gebildeten Korridor liegen muss. Vorteilhaft weist auch der zweite Grenzwert einen zeitlich veränderlichen Verlauf auf und ist besonders bevorzugt ebenfalls an den für die Strahlung charakteristischen zeitlichen Verlauf angepasst. Bei einem weiteren vorteilhaften Verfahren kann insbesondere aus einem Vergleich zwischen dem Verlauf und dem Grenzwert eine bestimmte Strahlungseinrichtung identifiziert werden. Insbesondere kann dabei eine solche Strahlungseinrichtung identifiziert werden, die für den Fehler verantwortlich ist. Dabei kann die Drehstellung des Transportrades bzw. der Transporteinrichtung verwendet werden.

Für die Innensterilisation der Behälter werden die Behälter auf einem Karussell behandelt. Mehrere Strahlenemitter sind auf diesem Karussell installiert und der Behälter wird relativ zum Emitter bewegt.

Im Fall einer Störung eines der Strahler verlässt das Messsignal den Toleranzkorridor und es wird eine Störung gemeldet. Aufgrund der Winkelabhängigkeit des Messsignals kann diese Störung auch einer bestimmten Station zugeordnet werden.

Auf diese Weise kann ein gealterter oder defekter Strahler auf Grund des Unterschreitens der Toleranzgrenze im Messsignal identifiziert werden. Der auf dieser Station behandelte Behälter kann ausgeschleust werden. Auch kann eine entsprechende Meldung an den Maschinenbediener erfolgen.

Im Fall einer deutlich überhöhten Strahlung kann ein Alarm ausgelöst und die Anlage in einen sicheren Zustand gebracht werden.

Die Amplitude dieses Signalhubs kann deutlich verstärkt werden, wenn der Sensor durch Abschattung oder Abschirmung nur Strahlung registriert, die aus einer bestimmten Richtung kommt. Je nach Karussellstellung sieht der Detektor dann maximales Signal, wenn sich ein Strahlfinger direkt davor befindet. Wenn kein Finger vor dem Detektor steht, sieht dieser nur einen diffusen Untergrund aus Streustrahlung.

Dieser diffuse Untergrund ist jedoch auch wichtig zu messen, er darf nicht zu schwach sein. Er muss noch deutlich höher sein, als wenn keine Strahlung herrscht. Dadurch wird detektiert, dass noch Strahlung in der Maschine herrscht. Die Abschirmung um den Detektor darf nicht zu stark sein.

Eine weitere Möglichkeit für die Behandlung der Behälter ist, ein oder mehrere Strahlenemitter fest an der Anlage (und nicht auf dem Karussell) zu installieren. Dies wird speziell zur Außensterilisation der Behälter angewandt, kann aber auch zur Innensterilisation verwendet werden.

Die Behälter fahren an diesem fest installierten Emitter vorbei und werden dabei bestrahlt. In diesem Fall kann der Sensor auf der gegenüberliegenden Seite des Emitters installiert werden, so dass er von den zu behandelnden Behältern abgeschattet wird. Durch das vorbeifahren der Behälter wird auch auf diese Weise ein zeitlich moduliertes, periodisches Signal auf dem Detektor erzeugt.

Die Sensoreinrichtung kann auch unterhalb der Preformen am Boden einer Rinne, innerhalb derer die Behältnisse transportiert werden, installiert werden und durch entsprechende Abschattung (z.B. eine sich an die Sensoreinrichtung anschließende lange Röhre) nur gerichtet nach oben auf die Bahn der Preformen blicken. Auf diese Weise sollte wieder ein maximaler Modulationshub erreicht werden.

Anhand der Form des Signals (Intensität und Dauer) kann im Vergleich mit einer angelernten Signalform entschieden werden, ob eine Störung vorliegt (z.B. dass ein Behälter in der Reihe fehlt, oder dass der Emitter zu starke oder zu schwache Strahlung aussendet.

Es können auch mehrere dieser Sensoren/Systeme in der Maschine verbaut werden, die jeweils eine unterschiedliche dynamische Signalform messen und auswerten. Die Quellen für die Röntgenstrahlung sind hier ebenfalls die Titanfolie des Emitters und die Behältnisse, insbesondere Vorformlinge, ggfs. auch deren Halterungen, die durch den Elektronenstrahl führen.

Bevorzugt soll aufgrund der Signalform entschieden werden, ob ein Prozess an einem einzelnen Behälter aus dem Rahmen fällt oder die Strahlung ein riskantes Limit übersteigt.

Bei den erwähnten Strahlungsdetektoreinrichtungen kann es sich um ein Dosimeter handeln, welches den Elektronenstrahl bzw. den Elektronenstrom bestimmt, es wäre jedoch auch möglich, Röntgendetektoren einzusetzen, welche die sich aus der Elektronenstrahlung ergebende Röntgenstrahlung messen.

Die vorliegende Erfindung ist weiterhin auf eine Vorrichtung zum Sterilisieren von Behältnissen gerichtet, welche eine Transporteinrichtung aufweist, welche die Behältnisse entlang eines vorgegebenen Transportpfades transportiert, sowie wenigstens eine erste Strahlungseinrichtung, welche wenigstens eine Wandung der Behältnisse mit einer Elektronenstrahlung beaufschlagt. Weiterhin weist die Vorrichtung eine Sensoreinrichtung auf, welche Elektronenstrahlung und/oder von den Elektronen verursachte Strahlung erfasst und bevorzugt ein Signal ausgibt, welches für wenigstens eine charakteristische Eigenschaft der auf die Sensoreinrichtung aufgetroffenen Strahlung charakteristisch ist (z.B. deren Intensität) und welche derart angeordnet ist, dass die auf die Sensoreinrichtung auftreffende Strahlung aufgrund der Bewegung der Behältnisse entlang des Transportpfades Schwankungen unterworfen ist.

Dabei erfasst die Sensoreinrichtung wenigstens einen für die Strahlung charakteristischen Werteverlauf und es ist weiterhin eine Vergleichseinrichtung vorgesehen, welche den charakteristischen Werteverlauf mit wenigstens einem ersten Grenzwert vergleicht. Erfindungsgemäß weist der Grenzwert einen zeitlich und/oder örtlich veränderlichen Wert auf.

Vorteilhaft handelt es sich bei der Transporteinrichtung um ein Rad, an dem auch eine Vielzahl von Halteeinrichtungen angeordnet ist, welche die Behältnisse während ihres Transportes halten. Bei diesen Halteeinrichtungen kann es sich beispielsweise um Greifklammern handeln, welche die Behältnisse an einer vorgegebenen Position, beispielsweise unterhalb ihres Tragringes greifen.

Bei einer weiteren vorteilhaften Ausführungsform weist die Vorrichtung mehrere Strahlungseinrichtungen auf, welche jeweils unterschiedliche Behältnisse bestrahlen. Vorteilhaft werden dabei die Strahlungseinrichtungen in die Behältnisse eingeführt. Vorteilhaft sind zusätzlich auch Bestrahlungseinrichtungen, d.H. Elektronenstrahler, vorgesehen, welche Außenwandungen der Behältnisse bestrahlen bzw. mit Elektronen beaufschlagen. Diese Bestrahlungseinrichtungen sind vorteilhaft stationär angeordnet.

Bei einer weiteren vorteilhaften Ausführungsform sind Bewegungseinrichtungen vorgesehen, welche die Behältnisse relativ zu den Strahlungseinrichtungen bewegen, so dass die Strahlungseinrichtungen zumindest teilweise über die Mündung der Behältnisse in diese eingeführt werden. Vorteilhaft bewegen dabei die Bewegungseinrichtungen die Behältnisse, wobei die Bewegungsrichtung vorteilhaft senkrecht zu einer Transportrichtung der Behältnisse entlang ihres Transportpfades ist.

Bei einer weiteren vorteilhaften Ausführungsform weist die Vorrichtung auch eine Positionserfassungseinrichtung auf, welche eine Position der Transporteinrichtung erfasst. Dabei kann es sich beispielsweise um einen Drehgeber handeln. Diese Drehgebersignale können ausgegeben werden, um auf diese Weise bei einer Vielzahl von Strahlungseinrichtungen eine bestimmte defekte Strahlungseinrichtung ermitteln zu können.

Weiterhin weist die Vorrichtung vorteilhaft einen Reinraum auf, innerhalb dessen die Sterilisation stattfindet. Dabei wäre es möglich, dass sowohl die Außensterilisation als auch die Innensterilisation der Behältnisse innerhalb dieses Reinraums stattfinden, es wäre jedoch auch möglich, dass einer der beiden Sterilisationsvorgänge außerhalb des Reinraums stattfindet.

Bei einer weiteren vorteilhaften Ausführungsform weist die Transporteinrichtung zum Transportieren wenigstens einen um eine Drehachse drehbaren Träger auf, an dem bevorzugt die Halteeinrichtungen zum Halten der Behältnisse angeordnet sind. Vorteilhaft weist die Transporteinrichtung wenigstens zwei drehbare Träger auf, wobei die Innensterilisation während des Transports der Behältnisse an dem ersten drehbaren Träger und die Außensterilisation der Behältnisse während des Transports der Behältnisse an dem zweiten drehbaren Träger vorgenommen wird.

Bei einer weiteren Ausführungsform weist die Vorrichtung auch wenigstens eine Abschirmungseinrichtung zum Abschirmen der entstehenden Strahlung wie insbesondere Röntgenstrahlung auf.

Weitere Vorteile und Ausführungen ergeben sich aus den beigefügten Zeichnungen: Darin zeigen:
- Fig. 1: eine Teilansicht einer erfindungsgemäßen Vorrichtung in einer ersten Ausführungsform;
- Fig. 2: eine weitere Ansicht einer erfindungsgemäßen Vorrichtung in einer zweiten Ausführungsform;
- Fig. 3 a-c: drei Darstellungen zur Veranschaulichung eines Sensorsignales und der dazugehörigen Grenzwerte;
- Fig. 4 a-c: drei Darstellungen zur Veranschaulichung einer möglichen Sensoranordnung;
- Fig. 5: eine Darstellung einer erfindungsgemäßen Anlage zum Sterilisieren von Behältnissen;
- Fig. 6: eine Darstellung zur Veranschaulichung der Anordnung der Sensoreinrichtungen bei der Innenbestrahlung; und
- Fig. 7: eine Darstellung zur Veranschaulichung der Anordnung der Sensoreinrichtungen bei der Außenbestrahlung.

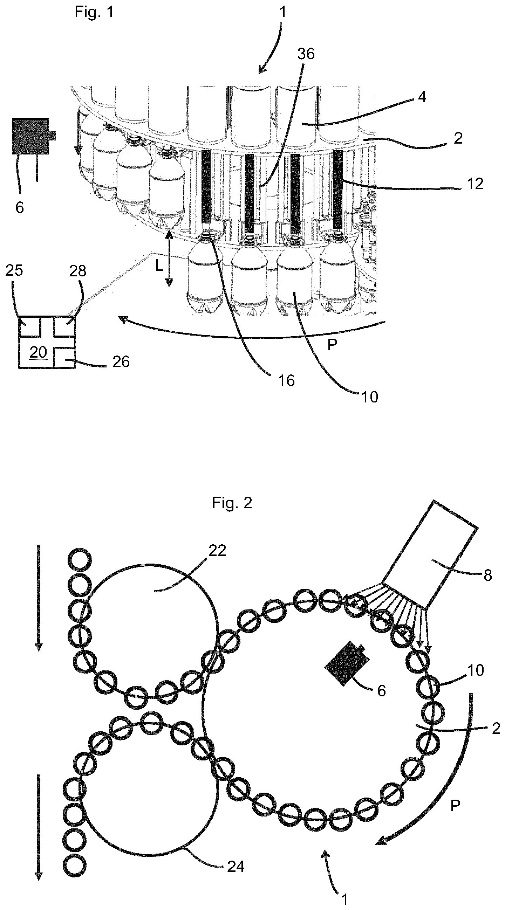

Fig. 1 zeigt eine Teilansicht einer erfindungsgemäßen Vorrichtung 1 zum Sterilisieren von Behältnissen 10. Dabei ist eine Transporteinrichtung 2 vorgesehen, wie beispielsweise ein Transportrad, an dem eine Vielzahl von Halteeinrichtungen 16 angeordnet ist, welche die Behältnisse 10 jeweils unterhalb ihres Tragringes greifen. Diese Halteeinrichtungen 16 sind dabei auch entlang ihrer Längsrichtung L mit einer Hubbewegung bewegbar, so dass die Strahlfinger 12, welche einen Bestandteil der in ihrer Gesamtheit mit 4 bezeichneten Strahlungseinrichtungen bilden, in die Behältnisse einführbar sind. Damit sind auch hier die Strahlungseinrichtungen 4 an dem Transportrad bzw. der Transporteinrichtung 2 angeordnet. Am unteren Ende der Strahlfinger weisen diese ein (nicht gezeigtes) Austrittsfenster auf, durch welches die Elektronen austreten und in den Innenraum der Behältnisse 10 eintreten können. Das Bezugszeichen P bezieht sich auf den Transportpfad der Behältnisse 10 bzw. die Drehrichtung der Transporteinrichtung 2.

Das Bezugszeichen 6 kennzeichnet einen Strahlungssensor, der hier Strahlung aufnimmt, hier insbesondere Röntgenstrahlung, wie sie aufgrund der Strahlungsemitter (bzw. der von diesen ausgesandten Elektronen) entsteht. Diese Strahlungsdetektoreinrichtung 6 ist hier stationär angeordnet und damit so angeordnet, dass zumindest zeitweise zwischen den Strahlungseinrichtungen 4, genauer, zwischen den Strahlfingern 12 und der Strahlungsdetektoreinrichtung 6 wenigstens eine Behältniswand der einzelnen Behältnisse 10 angeordnet ist.

Das Bezugszeichen 20 kennzeichnet eine Steuerungseinrichtung zum Steuern der einzelnen Strahlungseinrichtungen und beispielsweise auch der Hubbewegungen der Behältnisse und das Bezugszeichen 26 kennzeichnet (grob schematisch und nicht lagegetreu) einen Drehgeber, mit dessen Hilfe auch eine Drehstellung des Transportrades 2 erfasst werden kann.

Das Bezugszeichen 28 bezieht sich auf eine Vergleichseinrichtung, welche die von der Strahlungsdetektoreinrichtung 6 aufgenommenen Signale mit in einer Speichereinrichtung 25 abgespeicherten Grenzwerten vergleicht.

Es wäre dabei möglich, dass zunächst im Rahmen eines Einlernbetriebes die Strahlungsdaten aufgenommen werden bzw. ein für die Vorrichtung 1 typischer Strahlungsverlauf.

Figur 2 zeigt eine weitere Ausführungsform einer erfindungsgemäßen Vorrichtung. Bei dieser Ausführungsform ist eine stationär angeordnete Strahlungseinrichtung 4 vorgesehen, welche hier die Außenwandungen der Behältnisse 10 mit Elektronenstrahlung bestrahlt. Das Bezugszeichen 22 bezieht sich auf ein Zuführrad, welches die Behältnisse 10 der Vorrichtung 1 zuführt und das Bezugszeichen 24 kennzeichnet ein Abführrad, welches die im Außenbereich sterilisierten Behältnisse von der Vorrichtung abführt. Das Bezugszeichen P kennzeichnet hier wiederum den Transportpfad der Behältnisse. Damit steht die Strahlungseinrichtung hier fest und die Behältnisse 10 fahren an dieser vorbei. Durch den Abschattungseffekt der Behältnisse zwischen der Strahlungseinrichtung und der Strahlungserfassungseinrichtung 6 entsteht an dieser Strahlungsdetektoreinrichtung ein zeitlich moduliertes Signal.

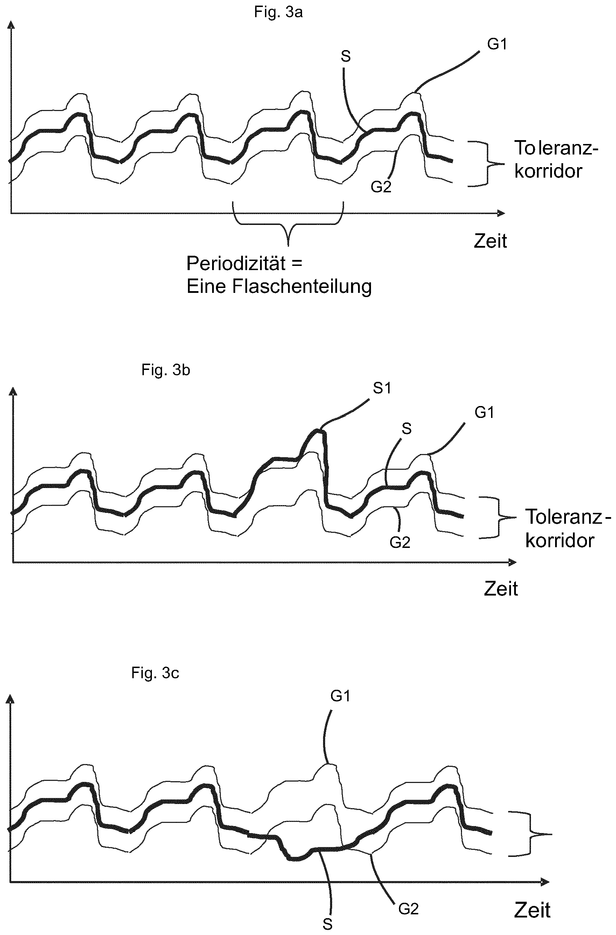

Die Figuren 3a-c veranschaulichen die jeweils aufgenommenen Signale bzw. die Grenzwertbildung. Dabei bezieht sich das Bezugszeichen S in allen drei Darstellungen jeweils auf das aufgenommene Sensorsignal. Dieses schwankt hier bei beiden in Figur 1 und 2 gezeigten Ausführungsformen periodisch, da sich im Falle der in Figur 1 gezeigten Ausführungsform die Strahlungseinrichtungen selbst an der Strahlungsdetektoreinrichtung vorbeibewegen und im Falle der in Figur 2 gezeigten Ausführungsform sich zumindest auch die Behältnisse selbst bewegen und sich damit die Beschattungseffekte periodisch ändern können.

Bei der in Figur 1 gezeigten Ausführungsform werden sich hinsichtlich des hier gezeigten Sensorsignals auch die einzelnen Perioden zumindest geringfügig unterscheiden, da diese jeweils den einzelnen Strahlungseinrichtungen, welche in die Behältnisse eintauchen, zuzuordnen sind.

Die Periodizität des Sensorsignals kennzeichnet damit bei beiden Ausführungsformen eine Flaschenteilung. Die Bezugszeichen G1 und G2 kennzeichnen obere und untere Grenzwerte, die hier, wie in den Figuren gezeigt, an das Sensorsignal angepasst sind. Dieses Anpassen kann in einem speziellen Einlernbetrieb erfolgen. Vorteilhaft werden hier die jeweiligen Strahlungswerte in Abhängigkeit von einer Drehstellung der Transporteinrichtung aufgenommen und damit die Grenzwertverläufe G1 und G2 beispielsweise durch Aufschläge und Abschläge von jeweils 10% bezüglich des Signalverlaufs gewählt. Die Strahlungswerte können dabei in Abhängigkeit von Drehgeberstellungen der Transporteinrichtung aufgenommen werden. Dabei wäre es möglich, diesen Verlauf mit einer vorgegebenen Anzahl an Stützstellen aufzunehmen. Zwischen diesen Stützstellen liegende Werte können beispielsweise durch Interpolation ermittelt werden. Auch wäre es möglich, die Strahlungsverläufe über mehrere Umläufe zu mitteln. Es wäre auch die Bildung von gleitenden Mittelwerten denkbar. Daneben wäre es jedoch auch möglich, den Verlauf der Grenzwerte etwa durch eine mathematische Funktion, etwa eine Sinusfunktion oder eine Rechtecksfunktion oder Kombinationen aus mehreren Funktionen anzugeben bzw. anzunähern. In diesem Falle könnte evtl. auf einen Einlernbetrieb verzichtet werden.

Das gemessene Sensorsignal muss sich daher zu jeder Zeit innerhalb des Toleranzkorridors, der zwischen diesen beiden Grenzwerten G1 und G2 gebildet wird, befinden. Bei der in Figur 3b gezeigten Darstellung weicht das Sensorsignal in einem Bereich S1 ab und überschreitet den oberen Grenzwert G1. Aus diesem Grunde kann darauf geschlossen werden, dass eine bestimmte Strahlungseinrichtung fehlerhaft ist. Bei diesem Überschreiten des Signals wäre es möglich, die Anlage unverzüglich abzuschalten. In diesem Falle verlässt das Sensorsignal S1 bei einem dritten Behälter den Toleranzkorridor, so dass darauf geschlossen werden kann, dass die entsprechende Strahlungseinrichtung defekt ist. Zur Kontrolle wäre es auch möglich, mehrere Perioden zu messen bzw. während mehrerer Volldrehungen der Transporteinrichtung. So könnte geprüft werden, ob das Signal jeweils derselben Strahlungseinrichtung mehrmals den Toleranzkorridor verlässt.

Bei der in Figur 3c gezeigten Ausgestaltung erkennt man, dass das Signal, welches hier ebenfalls einer bestimmten Strahlungseinrichtung zugeordnet ist, den unteren Grenzwert G2 unterschreitet. Auch hier kann darauf geschlossen werden, dass die entsprechende Strahlungseinrichtung nicht mehr (ausreichend) strahlt. Es wäre hier möglich, ebenfalls die Anlage abzuschalten und Reparaturmaßnahmen an der entsprechenden defekten Strahlungseinrichtung durchzuführen. Bevorzugt können im Rahmen von Reparaturmassnahmen ggfs. als defekt erkannte Strahler automatisch bzw. selbstständig in eine Serviceposition gebracht werden, beispielsweise in den Bereich einer Serviceöffnung.

Es wäre jedoch auch denkbar, vorübergehend die Anlage weiterlaufen zu lassen und sämtliche Behältnisse auszuschleusen, die von eben diesem defekten Strahler sterilisiert wurden. Ebenfalls ist es möglich, einlaufseitig nur die Positionen zu besetzen die ordnungsgemäß funktionierende Strahler aufweisen.

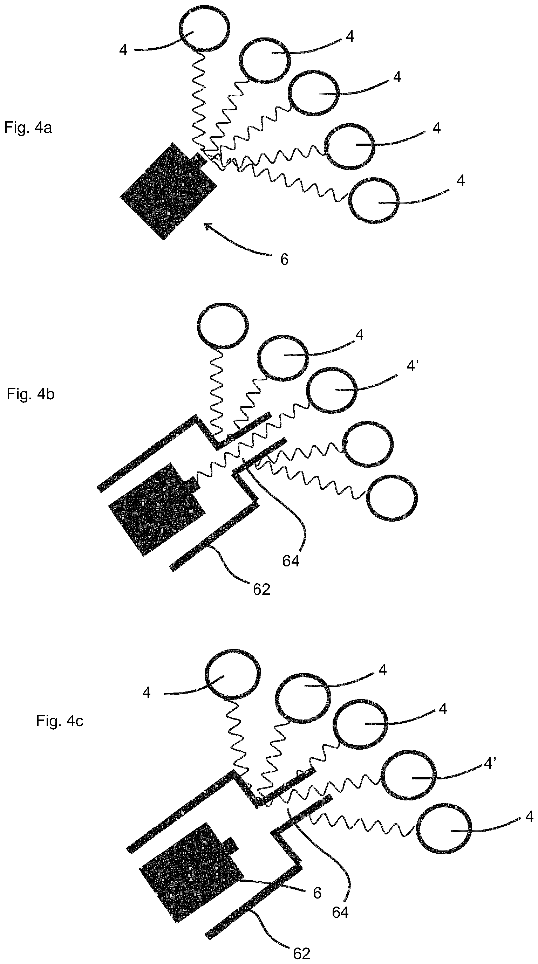

Die Figuren 4 a-c zeigen drei Ausgestaltungen für die Anordnungen der Strahlungsdetektoreinrichtung. Die Behältnisse sind hier nicht eingezeichnet. Bei der in Figur 4a gezeigten Situation erhält die Sensoreinrichtung gleichzeitig ein Signal aus verschiedenen Quellen. Dies führt zu einer diffusen Überlagerung, welche möglicherweise die Identifizierung einer bestimmten Strahlungseinrichtung erschwert. Bei der in Figur 4b gezeigten Situation ist ein Gehäuse 62 vorgesehen, welches eine Eintrittsöffnung 64 aufweist. Man erkennt, dass die Sensoreinrichtung in diesem Falle nur die Strahlung aus einer einzelnen Quelle 4' wahrnimmt und die anderen Strahlungen nicht bis zu der Sensoreinrichtung gelangen. Auf diese Weise wird zwar das gemessene Signal stärker moduliert sein, und es lässt sich damit eindeutig an jeder Position eine bestimmte gegebenenfalls defekte Strahlungseinrichtung zuordnen.

Bei der in Figur 4c gezeigten Situation erfasst die Sensoreinrichtung keine direkte Strahlung mehr, sondern nur noch diffusen Untergrund durch Streustrahlung 4, welche auf die Strahlungseinrichtung 4' zurückzuführen ist.

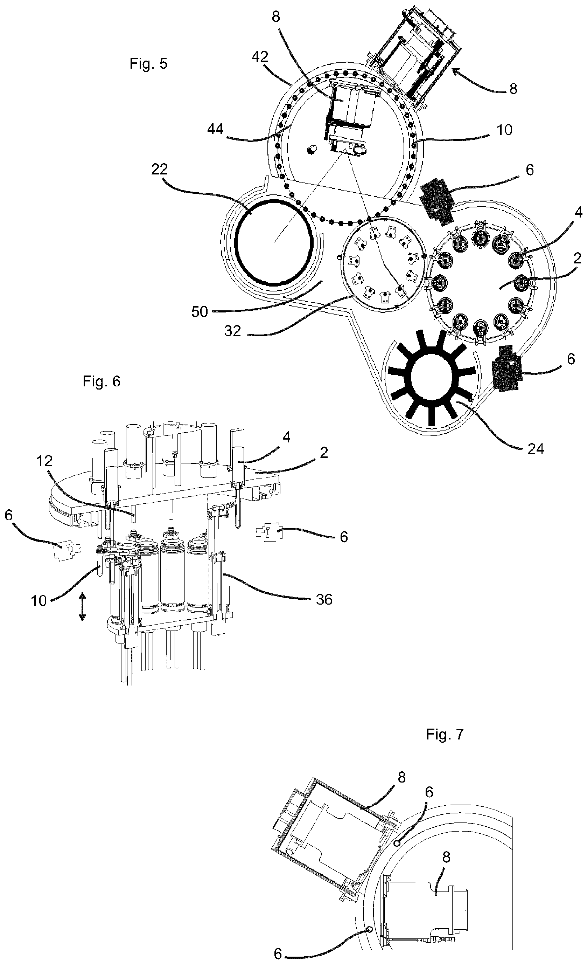

Fig. 5 zeigt eine Darstellung einer erfindungsgemäßen Anlage zum Sterilisieren von Behältnissen, hier von Kunststoffvorformlingen. Dabei werden die Kunststoffvorformlinge Transportiert und zunächst mit zwei Sterilisationseinrichtungen 8 an ihrer Aussenwandung sterilisiert. Die beiden Sterilisationseinrichtungen 8 sind hier auf gegenüberliegenden Seiten des Transportpfads sowie auch versetzt zueinander angeordnet. Die Bezugszeichen 42 und 44 kennzeichnen Wandungen, welche den Transportpfad der Behältnisse abschirmen. Diese Wandungen können dabei einerseits einen Reinraum begrenzen, innerhalb dessen die Behältnisse transportiert werden, andererseits jedoch auch zur Abschirmung der entstehenden Röntgenstrahlung dienen. Das Bezugszeichen 32 kennzeichnet einen Transferstern, der die Behältnisse von der Außensterilisation zu der Innensterilisation transportiert.

An die Außensterilisation der Behältnisse schließt sich damit eine Innensterilisation an, wobei hier, wie oben gezeigt, jeweils Strahlfinger in das Innere der Behältnisse eingeführt werden. Das Bezugszeichen 50 bezieht sich auf den Reinraum innerhalb dessen die Behältnisse sterilisiert werden.

Fig. 6 zeigt eine weitere Darstellung zur Veranschaulichung der Innensterilisation. Hier sind auch Hubeinrichtungen 36 gezeigt, welche die Behältnisse 10 anheben und so bewirken, dass die Strahlfinger 12 in die Behältnisse eingeführt werden.

Bevorzugt sind dabei Sensoreinrichtungen 6 im Bereich der Strahlungsfinger 12 platziert, besonders bevorzugt seitlich und besonders bevorzugt auf einer Höhe knapp unterhalb der Strahlaustrittsfenster. Die Sensoreinrichtungen sehen dann die Röntgenstrahlung der vorbeifahrenden Emitter. Aus sicherheitstechnischer Sicht ist es vorteilhaft, eine Sensoreinrichtung bzw. einen Detektor auf Basis eines Szintillators und eine Sensoreinrichtung auf Basis einer Ionisationskammer zu verwenden.

Die erste Sensoreinrichtung 6 (links in der Figur) nimmt das modulierte Signal auf und wertet aus, ob der Toleranzkorridor verlassen wird. Sie entscheidet am Beginn der Behandlung, ob der Preform bzw.das Behältnis ordnungsgemäß bestrahlt werden wird.

Die zweite stromabwärts bezüglich der ersten Sensorenrichtung angeordnete Sensoreinrichtung (rechts in der Figur) kann die Ergebnisse der ersten Sensoreinrichtung 6 bestätigen und detektiert zusätzlich, ob einer der Strahler während der Behandlung kaputt ging und ob es nötig wird, das entsprechende Behältnis 10 bzw. den entsprechenden Preform auszuschleusen. Hierbei wird unterstellt, dass die einzelnen Strahler zwischen diesen beiden Detektoren verwendungsgemäß funktionierten. Eine Störung aufgrund eines Arcs in einem Emitter wird so nicht festgestellt, dieser Fehler wird im Generator der Strahlungseinrichtung detektiert. Fig. 7 zeigt eine vergrößerte Darstellung zur Veranschaulichung der Außensterilisation der Behältnisse. Zwei Sensoreinrichtungen 6 (nur schematisch gezeigt) sind hier in dem Bereich der beiden Flächenstrahler 8 angeordnet. Dabei sind die Sensoreinrichtungen hier unterhalb des Transportpfads der Behältnisse 10 angeordnet.

Die Platzierung dieser Sensoreinrichtungen 6 könnte so erfolgen, dass beide Sensoreinrichtungen bzw. Detektoren jeweils hauptsächlich eine Strahlungsquelle sehen. Besonders bevorzugt sind sie derart abgeschattet (beispielsweise mittels einer Blende), dass sie nur dann ein starkes Signal bekommen, wenn ein Preform unmittelbar passiert. Sie sollen aber auch Streustrahlung der benachbarten Preformen und/oder des benachbarten Emitters sehen. Beide Detektoren sollten so ein ähnliches Signal erfahren.

Damit ist gewährleistet, dass man eine Störung der entsprechenden Preform oder Station zuordnen kann. Allerdings sehen beide Detektoren auch eine Untergrundstrahlung, wenn gerade kein Preform drüber steht. Die prinzipielle Funktion des Emitters wird damit bestätigt.

### Bezugszeichenliste

- 1: Vorrichtung
- 2: Transporteinrichtung/Transportrad
- 4: Strahlungseinrichtung für Innensterilisation
- 4': Strahlungseinrichtung
- 6: Strahlungsdetektoreinrichtung/Sensoreinrichtung
- 8: Strahlungseinrichtung für Außensterilisation
- 10: Behältnisse
- 12: Strahlfinger
- 16: Halteeinrichtungen
- 20: Steuerungseinrichtung
- 22: Zuführrad
- 24: Abführrad
- 25: Speichereinrichtung
- 26: Drehgeber
- 28: Vergleichseinrichtung
- 32: Transferstern
- 36: Hubeinrichtung
- 42, 44: Wandung
- 50: Reinraum
- 62: Gehäuse
- 64: Eintrittsöffnung

- G1, G2: Grenzwerte
- P: Transportpfad
- S, S1: Sensorsignal
- L: Längsrichtung der Behältnisse

## Patentansprüche

1. Verfahren zum Betreiben einer Vorrichtung (1) zum Sterilisieren von Behältnissen (10), wobei die Behältnisse (10) mittels einer Transporteinrichtung (2) entlang eines vorgegebenen Transportpfades (P) transportiert werden und während dieses Transports mittels wenigstens einer ersten Strahlungseinrichtung (4, 8) wenigstens eine Wandung der Behältnisse (10) mit einer Elektronenstrahlung bestrahlt wird, und wobei weiterhin eine Sensoreinrichtung (6) vorgesehen ist, welche Elektronenstrahlung und/oder von den Elektronen verursachte Strahlung erfasst und welche derart angeordnet ist, dass die auf die Sensoreinrichtung (6) auftreffende Strahlung aufgrund der Bewegung der Behältnisse (10) entlang des Transportpfades (P) Schwankungen unterworfen ist, und diese Sensoreinrichtung (6) wenigstens einen für die Strahlung charakteristischen Verlauf erfasst, wobei wenigstens ein erster Grenzwert (G1) definiert wird und ein Vergleich zwischen diesem Grenzwert (G1) und dem Verlauf durchgeführt wird
**dadurch gekennzeichnet, dass**
der Grenzwert (G1) einen zeitlich und/oder örtlich veränderlichen Wert aufweist, sodass anstatt einen konstanten Grenzwert festzulegen dieser sich ändert, und wobei somit der Grenzwert den zeitlichen und/oder ortsabhängigen Verlauf aufweist, der an den charakteristischen Verlauf der gemessenen Strahlung angepasst wird, und wobei daher die Anordnung der Sensoreinrichtung (6) bewusst so gewählt ist, dass von dieser kein konstantes Signal aufgenommen wird, sondern dieses bedingt durch die Bewegung der Behältnisse schwankt.

2. Verfahren nach wenigstens einem der vorangegangenen Ansprüche,
**dadurch gekennzeichnet, dass**
die Sensoreinrichtung derart angeordnet ist, dass zumindest Abschnitte der Behältnisse (10) zwischen der Strahlungseinrichtung (4) und der Sensoreinrichtung (6) transportiert werden.

3. Verfahren nach wenigstens einem der vorangegangenen Ansprüche,
**dadurch gekennzeichnet, dass**
durch den Vergleich zwischen dem Grenzwert und dem Verlauf ermittelt wird, ob die Bestrahlung der Behältnisse vorgegebenen Kriterien genügt.

4. Verfahren nach wenigstens einem der vorangegangenen Ansprüche,
**dadurch gekennzeichnet, dass**
wenigstens ein zweiter Grenzwert definiert wird und ein Vergleich zwischen diesem zweiten Grenzwert und dem Verlauf durchgeführt wird.

5. Verfahren nach wenigstens einem der vorangegangenen Ansprüchen,
**dadurch gekennzeichnet, dass**
die erste Strahlungseinrichtung (4) mit den Behältnissen (10) bewegt wird.

6. Verfahren nach wenigstens einem der vorangegangenen Ansprüche,
**dadurch gekennzeichnet, dass**
wenigstens ein Abschnitt der Strahlungseinrichtung (4) in einen Innenraum der Behältnisse (10) eingeführt wird

7. Verfahren nach Anspruch 1,
**dadurch gekennzeichnet, dass**
aus einem Vergleich zwischen dem Verlauf und dem Grenzwert eine bestimmte Strahlungseinrichtung identifiziert wird.

8. Vorrichtung (1) zum Sterilisieren von Behältnissen (10) mit einer Transporteinrichtung (2), welche die Behältnisse (10) entlang eines vorgegebenen Transportpfades (P) transportiert, mit wenigstens einer ersten Strahlungseinrichtung (4, 8), welche wenigstens eine Wandung der Behältnisse (10) mit einer Elektronenstrahlung beaufschlagt, mit einer Sensoreinrichtung (6), welche Elektronenstrahlung und/oder von den Elektronen verursachte Strahlung erfasst und welche derart angeordnet ist, dass die auf die Sensoreinrichtung (6) auftreffende Strahlung aufgrund der Bewegung der Behältnisse (10) entlang des Transportpfades (P) Schwankungen unterworfen ist, wobei diese Sensoreinrichtung (6) dazu eingerichtet und dafür vorgesehen ist wenigstens einen für die Strahlung charakteristischen Werteverlauf zu erfassen, und mit einer Vergleichseinrichtung, welche dazu eingerichtet und dafür vorgesehen ist den charakteristischen Werteverlauf mit wenigstens einem ersten Grenzwert (G1, G2) zu vergleichen,
**dadurch gekennzeichnet, dass**
der Grenzwert (G1) einen zeitlich und/oder örtlich veränderlichen Wert aufweist, sodass anstatt einen konstanten Grenzwert festzulegen, dieser sich ändert, und wobei somit der Grenzwert den zeitlichen und/oder ortsabhängigen Verlauf aufweist, der an den charakteristischen Verlauf der gemessenen Strahlung angepasst wird, und wobei daher die Anordnung der Sensoreinrichtung (6) bewusst so gewählt ist, dass von dieser kein konstantes Signal aufgenommen wird, sondern dieses bedingt durch die Bewegung der Behältnisse schwankt.

9. Vorrichtung (1) nach Anspruch 8,
**dadurch gekennzeichnet, dass**
die Vorrichtung (1) mehrere Strahlungseinrichtungen (4) aufweist, welche jeweils unterschiedliche Behältnisse (10) bestrahlen.

10. Vorrichtung nach wenigstens einem der vorangegangenen Ansprüche 9 - 10,
**dadurch gekennzeichnet, dass**
die Vorrichtung (1) eine Positionserfassungseinrichtung (26) aufweist, welche dazu eingerichtet und dafür vorgesehen ist eine Position der Transporteinrichtung (2) zu erfassen.

## Claims

1. A method of operating an apparatus (1) for the sterilization of containers (10), wherein the containers (10) are conveyed along a pre-set conveying path (P) by means of a conveying device (2) and during this conveying at least one wall of the containers (10) is irradiated with an electron radiation by means of at least one first radiation device (4, 8), and wherein furthermore a sensor device (6) is provided which detects electron radiation and/or radiation produced by the electrons and which is arranged in such a way that the radiation striking the sensor device (6) is subjected to fluctuations on account of the movement of the containers (10) along the conveying path (P), and this sensor device (6) detects at least one pattern characteristic of the radiation, wherein at least one first threshold value (G1) is defined and a comparison is made between this threshold value (G1) and the pattern,
**characterized in that**
the threshold value (G1) has a value which is variable in terms of time and/or location therefore, a constant threshold value is not set, but the threshold value varies and thus wherein the threshold value has the time and/or location-dependent pattern which is adapted to the characteristic pattern of the radiation measured and wherein the arrangement of the sensor device (6) is thus deliberately chosen in such a way that not a constant signal is received by it, but the aforesaid signal fluctuates as a result of the movement of the containers.

2. A method according to at least one of the preceding claims,
**characterized in that**
the sensor device is arranged in such a way that at least portions of the containers (10) are conveyed between the radiation device (4) and the sensor device (6).

3. A method according to at least one of the preceding claims,
**characterized in that**
by the comparison between the threshold value and the pattern it is determined whether the irradiation of the containers meets pre-set criteria.

4. A method according to at least one of the preceding claims,
**characterized in that**
at least one second threshold value is defined and a comparison is made between this second threshold value and the pattern.

5. A method according to at least one of the preceding claims,
**characterized in that**
the first radiation device (4) is moved with the containers (10).

6. A method according to at least one of the preceding claims,
**characterized in that**
at least one portion of the radiation device (4) is inserted into an interior of the containers (10).

7. A method according to claim 1,
**characterized in that**
a defined radiation device is identified from a comparison between the pattern and the threshold value.

8. An apparatus (1) for the sterilization of containers (10) with a conveying device (2) which conveys the containers (10) along a pre-set conveying path (P), with at least one first radiation device (4, 8) which acts upon at least one wall of the containers (10) with an electron radiation, with a sensor device (6) which detects electron radiation and/or radiation produced by the electrons and which is arranged in such a way that the radiation striking the sensor device (6) is subjected to fluctuations on account of the movement of the containers (10) along the conveying path (P), wherein this sensor device (6) is designed and intended to detect at least one value pattern characteristic of the radiation, and with a comparator device which is designed and intended to compare the characteristic value pattern with at least one first threshold value (G1, G2),
**characterized in that**
the threshold value (G1, G2) has a value which is variable in terms of time and/or location, therefore, a constant threshold value is not set, but the threshold value varies and thus wherein the threshold value has the time and/or location-dependent pattern which is adapted to the characteristic pattern of the radiation measured and wherein the arrangement of the sensor device (6) is thus deliberately chosen in such a way that a constant signal is not received by it, but the aforesaid signal fluctuates as a result of the movement of the containers.

9. An apparatus (1) according to claim 8,
**characterized in that**
the apparatus (1) has a plurality of radiation devices (4) which in each case irradiate different containers (10).

10. An apparatus according to at least one of the preceding claims 9 - 10,
**characterized in that**
the apparatus (1) has a position detection device (26) which is designed and intended to detect a position of the conveying device (2).

## Revendications

1. Procédé pour le fonctionnement d'un dispositif (1) de stérilisation de contenants (10), dans lequel les contenants (10) sont transportés le long d'un trajet de transport prédéterminé (P) au moyen d'un dispositif de transport (2) et pendant ce transport au moins une paroi des contenants (10) est irradiée par un rayonnement d'électrons au moyen d'au moins un premier dispositif de rayonnement (4, 8), et en outre, un dispositif capteur (6) est prévu qui détecte le rayonnement d'électrons et/ou le rayonnement provoqué par les électrons et qui est agencé de telle sorte que le rayonnement heurtant le dispositif capteur (6) en raison du mouvement des contenants (10) le long du le chemin de transport (P) est soumis à des fluctuations, et ce dispositif capteur (6) détecte au moins une courbe caractéristique du rayonnement, dans lequel au moins une première valeur limite (G1) est définie et une comparaison est effectuée entre cette valeur limite (G1) et la courbe,
**caractérisé en ce que**
la valeur limite (G1) a une valeur variable dans le temps et/ou dans l'espace, de sorte qu'au lieu de fixer une valeur limite constante, celle-ci change, et dans lequel la valeur limite a ainsi la courbe dépendante du temps et/ou de l'emplacement qui est adaptée à la courbe caractéristique du rayonnement mesuré, et l'agencement du dispositif capteur (6) est donc délibérément choisi pour qu'aucun signal constant ne soit enregistré par celui-ci, mais plutôt qu'il fluctue du fait du mouvement des contenants.

2. Procédé selon au moins l'une des revendications précédentes,
**caractérisé en ce que**
le dispositif capteur est agencé de telle sorte qu'au moins des sections des contenants (10) sont transportées entre le dispositif à rayonnement (4) et le dispositif capteur (6).

3. Procédé selon au moins l'une des revendications précédentes,
**caractérisé en ce que**
en comparant la valeur limite et la courbe, il est déterminé si l'irradiation des contenants répond aux critères spécifiés.

4. Procédé selon au moins l'une des revendications précédentes,
**caractérisé en ce que**
au moins une seconde valeur limite est définie et une comparaison est faite entre cette seconde valeur limite et la courbe.

5. Procédé selon au moins l'une des revendications précédentes,
**caractérisé en ce que**
le premier dispositif de rayonnement (4) est déplacé avec les contenants (10).

6. Procédé selon au moins l'une des revendications précédentes,
**caractérisé en ce que**
au moins une section du dispositif à rayonnement (4) est introduite dans un espace intérieur des contenants (10).

7. Procédé selon la revendication 1,
**caractérisé en ce que**
un appareil à rayonnement spécifique est identifié à partir d'une comparaison entre la courbe et la valeur limite.

8. Dispositif (1) de stérilisation de contenants (10) avec un dispositif de transport (2), qui transporte les contenants (10) le long d'un chemin de transport prédéterminé (P), avec au moins un premier dispositif de rayonnement (4, 8) qui expose au moins une paroi des contenants (10) à un rayonnement d'électrons, avec un dispositif capteur (6) qui détecte le rayonnement d'électrons et/ou le rayonnement provoqué par les électrons et qui est agencé de telle sorte que le rayonnement frappant le dispositif capteur (6) soit soumis à des fluctuations dues au mouvement des conteneurs (10) le long le chemin de transport (P), ce dispositif capteur (6) étant mis en place et prévu pour détecter au moins une courbe de valeur caractéristique du rayonnement, et avec un dispositif de comparaison qui est mis en place et prévu pour comparer la courbe de valeur caractéristique avec au moins une première valeur limite (G1, G2),
**caractérisé en ce que**
la valeur limite (G1) a une valeur variable temporelle et/ou spatiale, de sorte qu'au lieu de fixer une valeur limite constante, celle-ci change, et dans lequel la valeur limite a ainsi la courbe temporelle et/ou spatiale qui est adaptée à la courbe caractéristique du rayonnement mesuré, et l'agencement du dispositif capteur (6) est donc délibérément choisi pour qu'aucun signal constant ne soit enregistré par celui-ci, mais plutôt qu'il fluctue du fait du mouvement des récipients.

9. Dispositif (1) selon la revendication 8,
**caractérisé en ce que**
le dispositif (1) comporte une pluralité de dispositifs à rayonnement (4) qui irradient chacun différents contenants (10).

10. Dispositif selon au moins l'une des revendications 9 à 10,
**caractérisé en ce que**
le dispositif (1) comporte un dispositif de détection de position (26) qui est mis en place et prévu pour détecter une position du dispositif de transport (2).
